(19) Europäisches Patentamt
European Patent Office
Office européen des brevets

(11) **EP 1 959 914 B1**

(12) **EUROPEAN PATENT SPECIFICATION**

(45) Date of publication and mention
of the grant of the patent:
**21.05.2014 Bulletin 2014/21**

(21) Application number: **06818829.1**

(22) Date of filing: **27.11.2006**

(51) Int Cl.:
*A61K 8/11* (2006.01)          *A61K 8/29* (2006.01)
*A61K 8/02* (2006.01)          *A61K 8/25* (2006.01)
*A61K 8/35* (2006.01)          *A61K 8/891* (2006.01)
*A61K 8/36* (2006.01)          *A61Q 17/04* (2006.01)
*A61K 8/40* (2006.01)          *A61K 8/31* (2006.01)
*A61Q 19/00* (2006.01)        *A61K 8/34* (2006.01)

(86) International application number:
**PCT/EP2006/011326**

(87) International publication number:
**WO 2007/065574 (14.06.2007 Gazette 2007/24)**

(54) **COSMETIC OR DERMATOLOGICAL COMPOSITIONS COMPRISING MODIFIED TITANIUM DIOXIDE PARTICLES**

KOSMETISCHE ODER DERMATOLOGISCHE ZUSAMMENSETZUNGEN MIT MODIFIZIERTEN TITANDIOXID-TEILCHEN

NOUVELLES COMPOSITIONS COSMETIQUES OU DERMATOLOGIQUES COMPRENANT DES PARTICULES DE DIOXYDE DE TITANE MODIFIÉES

(84) Designated Contracting States:
**AT BE BG CH CY CZ DE DK EE ES FI FR GB GR HU IE IS IT LI LT LU LV MC NL PL PT RO SE SI SK TR**

(30) Priority: **09.12.2005 EP 05026944**

(43) Date of publication of application:
**27.08.2008 Bulletin 2008/35**

(73) Proprietor: **DSM IP Assets B.V.**
**6411 TE Heerlen (NL)**

(72) Inventors:
• **BERG-SCHULTZ, Katja**
**CH-4132 Muttenz (CH)**
• **MENDROK-EDINGER, Christine**
**79618 Rheinfelden-Minseln (DE)**
• **SIT, Fintan**
**229813 Singapore (SG)**
• **WESTENFELDER, Horst**
**67435 Neustadt a.d.W. (DE)**

(74) Representative: **Berg, Katja et al**
**DSM Nutritional Products Ltd**
**Patent Department**
**Wurmisweg 576**
**4303 Kaiseraugst (CH)**

(56) References cited:
**EP-A- 1 674 534          EP-A1- 0 748 624
WO-A2-02/22098          DE-A1- 19 750 030
FR-A- 2 855 751**

Remarks:
The file contains technical information submitted after the application was filed and not included in this specification

**Description**

The present invention relates to novel cosmetic or dermatological compositions comprising multiply coated titanium dioxide particles with a water content of less than 1.5%. More particularly, the present invention relates to novel cosmetic or dermatological compositions comprising multiply coated titanium dioxide particles with a water content of less than 1.5% and a dibenzoyl methane derivative.

There is a constantly increasing need for sunscreen protection agents in a population that is exposed to an increasing amount of damaging sunlight. Repetitive sun exposure can result in skin damages known as photoaged skin. The clinical changes that are seen in photoaged skin differ from those of normally aged skin in sunlight protected sites of the body. Among the damaging results of intensive sun exposure of the skin there is increased wrinkling, elastosis, pigmentary changes, precancerous and cancerous skin lesions.

Many sunscreening chemicals have been developed in the past protecting against the harmful effect of UV-A (320 to 400 nm) or UV-B (290-320 nm) radiation and even against radiation of shorter (UV-C) and longer wavelength (IR). Very recently a new class of sun screening chemicals, the broadband UV-filters have been developed which shield the skin from UV-A and UV-B radiation (290 to 400 nm). These chemicals are usually incorporated either alone or in combination with each other into cosmetic or dermatological preparations which are widely known and used. Preferable UV-B as well as UV-A sunscreening chemicals are present in a composition to protect human skin and/ or hair against harmful effects of UV radiation.

Within the class of UV-A filtering substances, dibenzoyl methane derivatives such as 4,4'-methoxy-tert.-butyl dibenzoylmethane (INCI: Butyl Methoxydibenzoylmethane) are especially interesting as they are widely used in the cosmetic industry. Such compounds are e.g. described in FR-A-2 326 405 and FR-A-2 440 933 and in EP-A-0114 607. 4,4'-Methoxy-tert.-butyl dibenzoylmethane is also known under the tradename PARSOL® 1789 by DSM Nutritional Products.

Dibenzoylmethane derivatives such as 4,4'-methoxy-tert.-butyl dibenzoylmethane are advantageously combined with UV-B sunscreening chemicals in order to obtain a protection over the whole UV range (290-400nm). Especially preferred is the combination of inorganic metal oxides, especially of titanium dioxide nanoparticles with 4,4'-methoxy-tert.-butyl dibenzoylmethane in order to obtain a broad sun protection activity of sunscreen composition and an efficient protection reflected by a high SPF.

Today, the combination of dibenzoylmethane derivatives, especially of 4,4'-methoxy-tert.-butyl dibenzoylmethane with commercially available titanium dioxide nanoparticles in cosmetic or dermatological compositions exhibits considerable disadvantages which negatively affect the quality and the acceptability of such products.

Cosmetic or dermatological compositions containing this combination often show a yellow to red discoloration which is highly undesirable. Additionally, an enhanced degradation of the dibenzoylmethane derivative in the presence of titanium dioxide nanoparticles is observed resulting in a loss of its efficacy as sun-screening agent. It is also known, that the addition of titanium dioxide nanoparticles leads to an increased tendency of crystallization of the dibenzoylmethane derivative resulting in an aesthetically unacceptable appearance as well as in a loss of the sun protection activity of the respective cosmetic or dermatological composition.

Titanium dioxide nanoparticles are also known to have photocatalytic activity through which reactions are triggered which may cause cell damage which is highly undesirable.

Various coatings have been proposed for modifying the surface of the titanium dioxide nanoparticles in order to avoid the incompatibilities with dibenzoylmethane, especially with 4,4'-methoxy-tert.-butyl dibenzoylmethane and at the same time to reduce the photocatalytic activity.

In particular in view of a reduction of the photocatalytic activity it has been suggested to use alumina coated titanium dioxide which are e.g. disclosed in EP 1 281 388. However, it is well known to a person skilled in the art that alumina coated titanium dioxide, with or without an additional surface treatment such as stearic acid, glycerol are not compatible with dibenzoylmethane derivatives such 4,4'-methoxy-tert. butyldibenzoylmethane.

EP-A 988 853, EP-A 1 284 277, EP0988853, and US 5562897, JP 2000319128 disclose silica coated metal oxide powders which claim to be compatible with dibenzoylmethane derivatives. However, besides showing a significant photocatalytic activity the compatibility with 4,4'-Methoxy-tert. butyldibenzoylmethane still remains a critical issue such as discoloration when incorporated together into cosmetic or dermatological compositions.

Document EP 748624 relates to UV protection cosmetic compositions comprising particulate titanium oxide having a silica layer, and optionally a further silicone layer. This document concerns improving the stability (and reducing the yellowing of) compositions comprising derivatives of dibenzoylmethane in combination with titanium oxide particles.

WO 0222098 relates to UV screening cosmetic compositions comprising surface treated particulate titanium and zinc oxides, resulting in compositions having good safety, stability and in-use sensory propertiels.

It is well known, that the incorporation of titanium dioxide particles into a cosmetic composition is often difficult as it is not easily wetted by water and/or a cosmetically acceptable solvent and consequently the dispersion remains a critical issue. Additionally, this leads to an uneven distribution of the active ingredient in the final product and thus in a reduction of its performance.

DE 103 33 029 A1 discloses silicium dioxide coated titanium dioxide nanoparticles and their use in UV protecting compositions in combination with dibenzoylmethane derivatives wherein the titanium dioxide has been hydrothermally treated. Such hydrothermal treated titanium dioxide nanoparticles have a water content of >1.8% and are thus not readily dispersible. The silicium dioxide coated titanium dioxide nanoparticles may also have a silicone or silane coating.

It has been found in accordance with the invention that multiply coated titanium dioxide particles wherein the titanium dioxide particles have not been hydrothermally treated and have a water content of less than 1.5% provide superior results in regard of dispersibility and consequently in their performance as sunscreening agent.

In accordance with the invention it has been found that multiply coated titanium dioxide particles having at least one inner inorganic silica coating and one outer organic coating and which have a water content of less than 1.5% (also referred to herein as double coated titanium dioxide) overcome the shortcomings of the prior art by showing at the same time an excellent dispersibility, a very low photocatalytic activity as well as an excellent compatibility with dibenzoylmethane derivatives. Therefore, such particles provide in combination with dibenzoylmethane derivatives in UV protecting compositions excellent results in regard of stability of the dibenzoylmethane derivative and UV protection of the entire composition.

Such double coated titanium dioxides nanoparticles typically comprise titanium dioxide which is obtainable according to the process as disclosed in EP 444798.

It has been found also that the UV protecting compositions according to the present invention provide significantly higher sun protection factors as corresponding compositions using other titanium dioxide grades coated with only one single coating (e.g. Uvinul® TiO$_2$ by BASF, Eusolex® T-Avo by Merck) and superior stabilization of the dibenzoylmethane derivative against degradation upon irradiation.

Additionally, it has been found that the UV protecting compositions according to the present invention show an enhanced transparency thus avoiding the so called 'whitening effect' on the skin.

Accordingly, in one aspect, the present invention is concerned with cosmetic or dermatological compositions comprising multiply coated titanium dioxide particles, said particles having at least one inner inorganic silica coating and one outer organic coating, and a water content of less than 1.5%, in particular less than 1.3%. Preferably, the cosmetic or dermatological compositions according to the invention also comprise additionally a dibenzoyl methane derivative, in particular 4,4'-methoxy-tert.-butyl dibenzoylmethane. Optionally, all cosmetic or dermatological compositions according to the invention may comprise further UV-A screening agent/s, and/ or UV-B screening agents and/ or broadband screening agent/s.

The invention also relates to the use of cosmetic or dermatological compositions according to the invention for the protection of human skin and/ or hair against UV-radiation.

In another aspect, the present invention is concerned with the use of the multiply coated titanium dioxide particles having at least one inner inorganic silica coating and one outer organic coating, and wherein the titanium dioxide has a water content of less than 1.5%, optionally in combination with an additional photostabilizer such as octocrylene, polysilicone-15, 4-methylbenzylidene camphor or mixtures thereof, for the stabilization of a dibenzoyl methane or a derivative thereof.

In a third aspect, the present invention is concerned with the use of multiply coated titanium dioxide particles having at least one inner inorganic silica coating and one outer organic coating, and wherein the titanium dioxide has a water content of less than 1.5%, for the enhancement of the sun protection factor (SPF) of cosmetic and dermatological compositions containing a dibenzoyl methane derivative.

The titanium dioxides nanoparticles can e.g. be prepared according to the process described in example 1 of EP 444798. The inner coating of the titanium dioxide particle with inorganic silica can be prepared according to the state of the art as e.g. described in EP 44515, EP-A 988 853, EP-A 1 284 277, EP0988853, and US 5562897, JP 2000319128. The inner coating layer consists of a minimum of 0.5 wt.% of inorganic silica (based on titanium dioxide). Preferably the inner coating layer consists of 0.5 wt.-% to 50 wt.-% most preferably of 1 wt.% to 20 wt.-% of inorganic silica (based on titanium dioxide). The outer coating can be selected from the class of organic coatings such as organic polymers e.g. silicone oils (e.g. simethicones, methicones, dimethicones, polysilicone-15), alkyl silanes, olefinic acids such as stearic acid or polyols such as glycerol or organophosphonic acids and mixtures thereof and can be applied to the titanium dioxide particle by methods known to a person skilled in the art e.g. described in FI57124. Preferably the outer coating is selected from simethicone, methicone, dimethicone, polysilicones-15, stearic acid, glycerol and mixtures thereof, most preferably of methicone, dimethicone, polysilicones-15 and/ or stearic acid in particular dimethicone. The outer coating layer consists of minimum 0.25 wt.-% based on titanium dioxide, preferably of 0.5 wt.-% to 50 wt.-% , most preferably of 0.5 wt.% to 10 wt.-%.

Multiple coated titanium dioxide nanoparticles prepared accordingly have a very low water content bound in it and thus exhibit a significantly better dispersibility.

The term "multiply coated" denotes the presence of at least two coatings on the titanium dioxide particles, i.e., an inner inorganic silica coating and an outer organic coating.

While in a preferred embodiment of the invention the UV protecting compositions comprise titanium dioxide particles having two coatings, i.e., an inner inorganic silica coating and an outer organic coating, other usual organic coatings can additionally be present. The other coatings can be applied before, after or together with the second outer coating. Other additional coatings which can be used are e.g. listed in EP 1 281 388 and comprise organic coatings such as stearic acid, silicones (silane derivatives such as triethoxycaprylylsilane or siloxane derivatives such as methicone, dimethicone, simethicone)

The surface of the titanium dioxide can be pretreated before the coating in order to additionally reduce the surface activity. Such pretreatments are well known to a person skilled in the art and can be performed e.g. with (a) fluoro acids selected from H2SiF6, $H_2TiF_6$, $H_2ZrF_6$, $H_2HfF_6$, $H_2GeF_6$, $H_2SnF_6$, and/or $HBF_4$; (b) water-sol. carboxylic acid containing ≥2 hydroxyl groups per carboxyl group in each acid mol.(esp. gluconic acid); (c) water-sol. salts of such carboxylic acids; (d) source of phosphate ions, esp. $H_3PO_4$ and/or phosphate salts and/ or organophosphoric acids and their salts; (e) inorganic acid such as $H_2SO_4$, $HNO_3$3, $H_3PO_4$, hydrobromic, hydroiodic and or perchloric acid (f) org. component selected from tannins and/or amino-phenolic polymers; and (h) optional oxide, hydroxide.

Preferably no pretreatment or a pretreatment with a source of phosphate ions, esp. $H_3PO_4$ and/or phosphate salts and/ or organophosphoric acids and their salts is applied.

All percentages and ratios mentioned in this specification are by weight if nothing else is stated or evident.

The water content of the titanium dioxide particles of this invention is to be understood as determined by Karl Fischer titration (e.g. described in Eugen Scholz Karl-Fischer-Titration, Springer-Verlag 1984 or the WHO Method WHO/M/7.R1).

The particle size of the titanium dioxide is not narrowly limited. All particle sizes which are principally useful for incorporating into sunscreen compositions can be used in cosmetic or dermatological compositions according to the present invention. However, the primary particle size of the double coated titanium dioxide is preferably in the range from 2 to 100 nm, more preferably in the range of 5 to 50 nm and the secondary particle size is preferably between 0.05 and 50 $\mu$m, more -preferably between 0.1 and 1 $\mu$m.

Cosmetic or dermatological compositions according to the present invention containing the double coated titanium dioxide, optionally in combination with a dibenzoylmethane and further optionally in combination with further UV-A screening agent/s, and/ or UV-B screening agents and/ or broadband screening agent/s, can be prepared according to the state in the art such as described in Novak G.A., Die kosmetischen Präparate - Band 2, Die kosmetischen Präparate - Rezeptur, Rohstoffe, wissenschaftliche Grundlagen (Verlag für Chem. Industrie H. Ziolkowski KG, Augsburg).

Preferred are cosmetic or dermatological compositions for the protection of the skin against UV-radiation such as topical sunscreen compositions.

The cosmetic or dermatological compositions contain the double coated titanium in an effective amount. The term "effective amount" means generally at least 0.5% by weight of the composition.

Preferred according to the inventions are cosmetic or dermatological compositions containing dibenzoylmethane derivatives and a double coated titanium dioxide wherein the outer coating consists of methicone, dimethicone, stearic acid or mixtures thereof. Most preferred according to the invention are cosmetic or dermatological compositions containing 4,4'-Methoxy-tert.butyldibenzoylmethane (PARSOL® 1789) and a double coated titanium dioxide wherein the outer coating of the particle is dimethicone. Preferably, the cosmetic or dermatological compositions according to the invention contain from 1 wt.% to 25 wt.-% of the multiply coated titanium dioxide and 0.5 wt.-% to 7 wt.-% of the dibenzoylmethane derivative, in particular 4,4'-Methoxy-tert.butyldibenzoylmethane (PARSOL® 1789) based on the total weight of the composition.

For the preparation of the topical sunscreen compositions, especially preparations for dermatological and/or cosmetic use, such as skin protection and sunscreen compositions for everyday cosmetics the double coated titanium dioxide and the dibenzoyl methane derivative can be incorporated in auxiliary agents, e.g. a cosmetic base, which are conventionally used for such compositions. Where convenient, other conventional UV-A and/or UV-B and/ or broad spectrum screening agents may also be added. The combination of UV screens may show a synergistic effect. The amount of the double coated titanium dioxide and of the dibenzoyl methane derivative and optionally other known UV-screens is not critical. Suitable amounts of the double coated titanium dioxide according to present invention are about 0.5 wt.-% to about 50% wt.-%, preferably about 1 wt.-% to 25 wt.-%. Suitable amounts of the dibenzoyl methane derivative to be combined with the double coated titanium dioxide is about 0.5 wt.% to 12 wt.%, preferably 1 wt.-% to 5 wt.%. Optionally about 0.5 wt.% to 12 wt.% by weight of additional, hydrophilic and/or lipophilic UV-A or UV-B or broad spectrum screening agent may be present. These additional screening agents are advantageously selected from among the compounds listed below without being limited thereto:

Examples of UV B or broad spectrum screening agents, i.e. substances having absorption maximums between about 290 and 340 nm, which come into consideration for combination with the coated particles of the present invention are for example the following organic and inorganic compounds: Acrylates such as 2-ethylhexyl 2-cyano-3,3-diphenylacrylate (octocrylene, PARSOL® 340), ethyl 2-cyano-3,3-diphenylacrylate and the like; Camphor derivatives such as 4-methyl benzylidene camphor (PARSOL® 5000), 3-benzylidene camphor, camphor benzalkonium methosulfate, polyacrylamidomethyl benzylidene camphor, sulfo benzylidene camphor, sulphomethyl benzylidene camphor, therephthalidene dicamphor sulfonic acid and the like; Cinnamate derivatives such as ethylhexyl methoxycinnamate (PARSOL® MCX), ethoxyethyl methoxycinnamate, diethanolamine methoxycinnamate (PARSOL® Hydro), isoamyl methoxycinnamate and the like as well as cinnamic acid derivatives bond to siloxanes; p-Aminobenzoic acid derivatives, such as p-aminobenzoic acid, 2-ethylhexyl p-dimethylaminobenzoate, N-oxypropylenated ethyl p-aminobenzoate, glyceryl p-aminobenzoate, Benzophenones such as benzophenone-3, benzophenone-4, 2,2', 4, 4'-tetrahydroxy-benzophenone, 2,2'-dihydroxy-4,4'-dimethoxy-benzophenone and the like; Esters of Benzalmalonic acid such as di-(2-ethylhexyl) 4-methoxybenzalmalonate; Esters of 2-(4-ethoxy-anilinomethylene)propandioic acid such as 2-(4-ethoxy anilinomethylene)propandioic acid diethyl ester as described in the European Patent Publication EP 0895 776; Organosiloxane, compounds containing benzmalonate groups as described in the European Patent Publications EP 0358584 B1, EP 0538431 B1 and EP 0709080 A1 such as polysilicones-15 (PARSOL® SLX); Drometrizole trisiloxane (Mexoryl XL); Pigments such as microparticulated $TiO_2$, and the like. The term "microparticulated" refers to a particle size from about 5 nm to about 200 nm, particularly from about 15 nm to about 100 nm. The $TiO_2$ particles may also be coated by metal oxides such as e.g. aluminum or zirconium oxides or by organic coatings such as e.g. polyols, methicone, aluminum stearate, alkyl silane. Such coatings are well known in the art. Imidazole derivatives such as e.g. 2-phenyl benzimidazole sulfonic acid and its salts (PARSOL®HS). Salts of 2-phenyl benzimidazole sulfonic acid are e.g. alkali salts such as sodium- or potassium salts, ammonium salts, morpholine salts, salts of primary, sec. and tert. amines like monoethanolamine salts, diethanolamine salts and the like. Salicylate derivatives such as isopropylbenzyl salicylate, benzyl salicylate, butyl salicylate, octyl salicylate (NEO HELIOPAN OS), isooctyl salicylate or homomenthyl salicylate (homosalate, HELIOPAN) and the like; Triazine derivatives such as octyl triazone (UVINUL T-150), dioctyl butamido triazone (UVASORB® HEB), bis ethoxyphenol methoxyphenyl triazine (Tinosorb® S) and the like; Encapsulated UV-filters such as encapsulated octyl methoxycinnamate (Eusolex® UV-pearls) and the like.

Examples of broad spectrum or UV A screening agents i.e. substances having absorption maximums between about 320 and 400 nm, which come into consideration as additional components in the compositions of the present invention are for example the following organic and inorganic compounds: Benzotriazole derivatives such as 2,2'-methylene-bis-(6-(2H-benzotriazole-2-yl)-4-(1,1,3,3,-tetramethylbutyl)-phenol (TINOSORB® M) and the like; Phenylene-1,4-bis-benzimidazolsulfonic acids or salts such as 2,2-(1,4-phenylene)bis-(1H-benzimidazol-4,6-disulfonic acid) (Neo heliopan® AP); Amino substituted hydroxybenzophenones such as 2-(4-Diethylamino-2-hydroxy-benzoyl)-benzoic acid hexylester (Uvinul®A Plus) as described in the European Patent Publication EP 1046391. Pigments such as microparticulated ZnO or $TiO_2$ and the like. The term "microparticulated" refers to a particle size from about 5 nm to about 200 nm, particularly from about 15 nm to about 100 nm. The particles may also be coated by other metal oxides such as e.g. aluminum or zirconium oxides or by organic coatings such as e.g. polyols, methicone, aluminum stearate, alkyl silane. Such coatings are well known in the art.

In a preferred embodiment of the invention the cosmetic or dermatological compositions comprising a dibenzoylmethane derivative, in particular 4,4'-Methoxy-tert.butyldibenzoylmethane contain an additional photostabilizer. Suitable photostabilizer for 4,4'-Methoxy-tert.butyldibenzoylmethane are e.g. 3,3-Diphenylacrylate derivatives as described in the EP 0 514 491 B1 and EP 0 780 119 A1; Benzylidene camphor derivatives as described in the US Patent No. 5,605,680; Organosiloxanes containing benzmalonate groups as described EP 0358584 B1, EP 0538431 B1 and EP 0709080 A1 or mixtures thereof. Preferably the photostabilizer is selected from polysilicones-15, octocrylene, 4-methylbenzylidede camphor or mixtures thereof. The amount of photostabilizer is not critical. The compositions contain the photostabilizer in an effective amount. The term "effective amount" means generally at least 0.1 wt.-% by weight of the light screening composition. Preferably, the compositions contain the photostabilizer in an amount of 0.1 wt.-% to 30 wt.%, more preferably 0.5 wt.-% to 20 wt.%, still more preferably 1 wt.-% to 10 wt.-%.

The compositions of the invention can also contain usual cosmetic adjuvants and additives, such as preservatives/antioxidants, fatty substances/oils, water, organic solvents, silicones, thickeners, softeners, emulsifiers, additional sunscreens, antifoaming agents, moisturizers, fragrances, surfactants, fillers, sequestering agents, anionic, cationic, nonionic or amphoteric polymers or mixtures thereof, propellants, acidifying or basifying agents, dyes, colorants, pigments or nanopigments, in particular those suited for providing an additional photoprotective effect by physically blocking out ultraviolet radiation, or any other ingredients usually formulated into cosmetics, in particular for the production of sunscreen/antisun compositions. The necessary amounts of the cosmetic and dermatological adjuvants and additives can, based on the desired product, easily be chosen by a skilled artisan in this field and will be illustrated in the examples, without being limited hereto. The usual cosmetic adjuvants and additives such as emulsifiers, thickeners, surface active ingredients and film formers can show synergistic effects in regard to the sun protection factors.

An additional amount of antioxidants/preservatives is generally preferred. Based on the invention all known antioxidants usually formulated into cosmetics can be used. Especially preferred are antioxidants chosen from the group consisting of amino acids (e.g. glycine, histidine, tyrosine, tryptophan) and their derivatives, imidazole (e.g. urocanic acid) and derivatives, peptides such as D,L-carnosine, D-carnosine, L-carnosine and derivatives (e.g. anserine), carotenoids, carotenes (e.g. α-carotene, β-carotene, lycopene) and derivatives, chlorogenic acid and derivatives, lipoic acid and derivatives (e.g. dihydrolipoic acid), aurothioglucose, propylthiouracil and other thiols (e.g. thioredoxine, glutathione, cysteine, cystine, cystamine and its glycosyl-, N-acetyl-, methyl-, ethyl-, propyl-, amyl-, butyl- and lauryl-, palmitoyl-; oleyl-, γ-linoleyl-, cholesteryl- and glycerylester) and the salts thereof, dilaurylthiodipropionate, distearylthiodipropionate, thiodipropionic acid and its derivatives (ester, ether, peptides, lipids, nucleotides, nucleosides and salts) as well as sulfoximine compounds (such as buthioninsulfoximine, homocysteinesulfoximine, buthioninsulfone, penta-, hexa-, heptathioninsulfoximine) in very low compatible doses (e.g. pmol bis μmol/kg), additionally (metal)-chelators (such as α-hydroxyfatty acids, palmic-, phytinic acid, lactoferrin), β-hydroxyacids (such as citric acid, lactic acid, malic acid), huminic acid, gallic acid, gallic extracts, bilirubin, biliverdin, EDTA, EGTA and its derivatives, unsaturated fatty acids and their derivatives (such as γ-linoleic acid, linolic acid, oleic acid), folic acid and its derivatives, ubiquinone and ubiquinol and their derivatives, vitamin C and derivatives (such as ascorbylpalmitate and ascorbyltetraisopalmitate, Mg-ascorbylphosphate, Na-ascorbylphosphate, ascorbyl-acetate), tocopherol and derivates (such as vitamin-E-acetate), mixtures of nat. vitamin E, vitamin A and derivatives (vitamin-A-palmitate and -acetate) as well as coniferylbenzoate, rutinic acid and derivatives, α-glycosylrutin, ferulic acid, furfurylideneglucitol, carnosine, butylhydroxytoluene, butylhydroxyanisole, trihydroxybutyrophenone, urea and its derivatives, mannose and derivatives, zinc and derivatives (e.g. ZnO, $ZnSO_4$), selen and derivatives (e.g. selenomethionin), stilbenes and derivatives (such as stilbenoxide, transstilbenoxide) and suitable derivatives (salts, esters, ethers, sugars, nucleotides, nucleosides, peptides and lipids) of the named active ingredients. One or more preservatives/antioxidants may be present in an amount about 0.01 wt.-% to about 10 wt.-% of the total weight of the composition of the present invention. Preferably, one or more preservatives/antioxidants are present in an amount about 0.1 wt.-% to about 1 wt.%.

Typically compositions also contain surface active ingredients like emulsifiers, solubilizers and the like. An emulsifier enables two or more immiscible components to be combined homogeneously. Moreover, the emulsifier acts to stabilize the composition. Emulsifiers that may be used in the present invention in order to form O/W, W/O, O/W/O or W/O/W emulsions/ microemulsions include sorbitan oleate, sorbitan sesquioleate, sorbitan isostearate, sorbitan trioleate, polyglyceryl-3-diisostearate, polyglycerol esters of oleic/isostearic acid, polyglyceryl-6 hexaricinolate, polyglyceryl-4-oleate, polygylceryl-4 oleate/PEG-8 propylene glycol cocoate, oleamide DEA, TEA myristate, TEA stearate, magnesium stearate, sodium stearate, potassium laurate, potassium ricinoleate, sodium cocoate, sodium tallowate, potassium castorate, sodium oleate, and mixtures thereof. Further suitable emulsifiers are phosphate esters and the salts thereof such as cetyl phosphate (Amphisol® A), diethanolamine cetyl phosphate (Amphisol®), potassium cetyl phosphate (Amphisol® K), sodium glyceryl oleate phosphate, hydrogenated vegetable glycerides phosphate and mixtures thereof. Furthermore, one or more synthetic polymers may be used as an emulsifier. For example, PVP eicosene copolymer, acrylates/$C_{10-30}$ alkyl acrylate crosspolymer, acrylates/steareth-20 methacrylate copolymer, PEG-22/dodecyl glycol copolymer, PEG-45/dodecyl glycol copolymer, and mixtures thereof. The preferred emulsifiers are cetyl phosphate (Amphisol® A), diethanolamine cetyl phosphate (Amphisol®), potassium cetyl phosphate (Amphisol® K), PVP Eicosene copolymer, acrylates/$C_{10-30}$-alkyl acrylate crosspolymer, PEG-20 sorbitan isostearate, sorbitan isostearate, and mixtures thereof. The one or more emulsifiers are present in a total amount about 0.01 wt.-% to about 20 wt.-% of the total weight of the composition of the present invention. Preferably, about 0.1 wt.-% to about 10 wt.-% of emulsifiers are used.

The lipid phase can advantageously be chosen from: mineral oils and mineral waxes; oils such as triglycerides of caprinic acid or caprylic acid, preferable castor oil; oils or waxes and other natural or synthetic oils, in an preferred embodiment esters of fatty acids with alcohols e.g. isopropanol, propyleneglycol, glycerin or esters of fatty alcohols with carbonic acids or fatty acids; alkylbenzoates; and/or silicone oils such as dimethylpolysiloxane, diethylpolysiloxane, diphenylpolysiloxane, cyclomethicones and mixtures thereof.

Exemplary fatty substances which can be incorporated in the oil phase of the emulsion, microemulsion, oleo gel, hydrodispersion or lipodispersion of the present invention are advantageously chosen from esters of saturated and/or unsaturated, linear or branched alkyl carboxylic acids with 3 to 30 carbon atoms, and saturated and/or unsaturated, linear and/or branched alcohols with 3 to 30 carbon atoms as well as esters of aromatic carboxylic acids and of saturated and/or unsaturated, linear or branched alcohols of 3-30 carbon atoms. Such esters can advantageously be selected from octylpalmitate, octylcocoate, octylisostearate, octyldodecylmyristate, cetearylisononanoate, isopropylmyristate, isopropylpalmitate, isopropylstearate, isopropyloleate, n-butylstearate, n-hexyllaureate, n-decyloleate, isooctylstearate, isononylstearate, isononylisononanoate, 2- ethyl hexylpalmitate, 2-ethylhexyllaurate, 2-hexyldecylstearate, 2-octyldodecylpalmitate, stearylheptanoate, oleyloleate, oleylerucate, erucyloleate, erucylerucate, tridecylstearate, tridecyltrimellitate, as well as synthetic, half-synthetic or natural mixtures of such esters e.g. jojoba oil.

Other fatty components suitable for use in the composition of the present invention include polar oils such as lecithins and fatty acid triglycerides, namely triglycerol esters of saturated and/or unsaturated, straight or branched carboxylic acid with 8 to 24 carbon atoms, preferably of 12 to 18 carbon-atoms whereas the fatty acid triglycerides are preferably chosen from synthetic, half synthetic or natural oils (e.g. cocoglyceride, olive oil, sun flower oil, soybean oil, peanut oil, rape seed oil, sweet almond oil, palm oil, coconut oil, castor oil, hydrogenated castor oil, wheat oil, grape seed oil, macadamia nut oil and others); apolar oils such as linear and/ or branched hydrocarbons and waxes e.g. mineral oils, vaseline (petrolatum); paraffins, squalane and squalene, polyolefins, hydrogenated polyisobutenes and isohexadecanes, favored polyolefins are polydecenes; dialkyl ethers such as dicaprylylether; linear or cyclic silicone oils such as preferably cyclomethicone (octamethylcyclotetrasiloxane; cetyldimethicone, hexamethylcyclotrisiloxane, polydimethylsiloxane, poly(methylphenylsiloxane) and mixtures thereof.

Other fatty components which can advantageously be incorporated in compositions of the present invention are isoeikosane; neopentylglycoldiheptanoate; propyleneglycoldicaprylate/ dicaprate; caprylic/ capric/ diglycerylsuccinate; butyleneglycol caprylat/caprat; $C_{12-13}$-alkyllactate; di-$C_{12-13}$-alkyltartrate; triisostearin; dipentaerythrityl hexacaprylat/hexacaprate; propyleneglycolmonoisostearate; tricaprylin; dimethylisosorbid. Especially beneficial is the use of mixtures $C_{12-15}$-alkylbenzoate and 2-ethylhexylisostearate, mixtures $C_{12-15}$-alkylbenzoate and isotridecylisononanoate as well as mixtures of $C_{12-15}$-alkylbenzoate, 2-ethythexylisostearate and isotridecylisononanoate.

The oily phase of the composition of the present invention can also contain natural vegetable or animal waxes such as bee wax, china wax, bumblebee wax and other waxes of insects as well as shea butter and cocoa butter.

A moisturizing agent may be incorporated into a composition of the present invention to maintain hydration or rehydrate the skin. Moisturizers that prevent water from evaporating from the skin by providing a protective coating are called emollients. Additionally an emollient provides a softening or soothing effect on the skin surface and is generally considered safe for topical use. Preferred emollients include mineral oils, lanolin, petrolatum, capric/caprylic triglyceraldehydes, cholesterol, silicones such as dimeticone, cyclometicone, almond oil, jojoba oil, avocado oil, castor oil, sesame oil, sunflower oil, coconut oil and grape seed oil, cocoa butter, olive oil aloe extracts, fatty acids such as oleic and stearic, fatty alcohols such as cetyl and hexadecyl (ENJAY), diisopropyl adipate, hydroxybenzoate esters, benzoic acid esters of $C_{9-15}$-alcohols, isononyl iso-nonanoate, ethers such as polyoxypropylene butyl ethers and polyoxypropylene cetyl ethers, and $C_{12-15}$-alkyl benzoates, and mixtures thereof. The most preferred emollients are hydroxybenzoate esters, aloe vera, $C_{12-15}$-alkyl benzoates, and mixtures thereof. An emollient is present in an amount of about 1 wt.-% to about 20 wt.% of the total weight of the composition. The preferred amount of emollient is about 2 wt.-% to about 15 wt.%, and most preferably about 4 wt.-% to about 10 wt.%.

Moisturizers that bind water, thereby retaining it on the skin surface are called humectants. Suitable humectants can be incorporated into a composition of the present invention such as glycerin, polypropylene glycol, polyethylene glycol, lactic acid, pyrrolidone carboxylic acid, urea, phospholipids, collagen, elastin, ceramides, lecithin sorbitol, PEG-4, and mixtures thereof. Additional suitable moisturizers are polymeric moisturizers of the family of water soluble and/ or swellable/ and/ or with water gelating polysaccharides such as hyaluronic acid, chitosan and/or a fucose rich polysaccharide which is e.g. available as Fucogel®1000 (CAS-Nr. 178463-23-5) by SOLABIA S. One or more humectants are optionally present at about 0.5 wt.-% to about 8 wt.% in a composition of the present invention, preferably about 1 wt.-% to about 5 wt.%.

The aqueous phase of the compositions of the present invention can contain the usual cosmetic additives such as alcohols, especially lower alcohols, preferably ethanol and/ or isopropanol, low diols or polyols and their ethers, preferably propyleneglycol, glycerin, ethyleneglycol, ethyleneglycol monoethyl- or monobutylether, propyleneglycol monomethyl-or -monoethyl- or-monobutylether, diethyleneglycol monomethyl-or monoethylether and analogue products, polymers, foam stabilizers; electrolytes and especially one or more thickeners. Thickeners that may be used in compositions of the present invention to assist in making the consistency of a product suitable include carbomer, siliciumdioxide, magnesium and/ or aluminum silicates, beeswax, stearic acid, stearyl alcohol polysaccharides and their derivatives such as xanthan gum, hydroxypropyl cellulose, polyacrylamides, acrylate crosspolymers preferably a carbomer, such as carbopole® of type 980, 981, 1382, 2984, 5984 alone or mixtures thereof. Suitable neutralizing agents which may be included in the composition of the present invention to neutralize components such as e.g. an emulsifier or a foam builder/stabilizer include but are not limited to alkali hydroxides such as a sodium and potassium hydroxide; organic bases such as diethanolamine (DEA), triethanolamine (TEA), aminomethyl propanol, and mixtures thereof; amino acids such as arginine and lysine and any combination of any foregoing. The neutralizing agent can be present in an amount of about 0.01 wt.-% to about 8 wt.% in the composition of the present invention, preferably, 1 wt.% to about 5 wt.%.

The addition of electrolytes into the composition of the present invention may be necessary to change the behavior of a hydrophobic emulsifier. Thus, the emulsions/ microemulsions of this invention may contain preferably electrolytes of one or several salts including anions such as chloride, sulfates, carbonate, borate and aluminate, without being limited thereto. Other suitable electrolytes can be on the basis of organic anions such as, but not limited to, lactate, acetate, benzoate, propionate, tartrate and citrate. As cations preferably ammonium, alkylammonium, alkali- or alkaline earth metals, magnesium-, iron- or zinc-ions are selected. Especially preferred salts are potassium and sodium chloride, magnesium sulfate, zinc sulfate and mixtures thereof. Electrolytes can be present in an amount of about 0.01 wt.-% to about 8 wt.-% in the composition of the present invention.

The cosmetic compositions of the invention are useful as compositions for photoprotecting the human epidermis or hair against the damaging effect of ultraviolet irradiation, as sunscreen compositions. Such compositions can, in particular, be provided in the form of a lotion, a thickened lotion, a gel, a cream, a milk, an ointment, a powder or a solid tube stick and can be optionally be packaged as an aerosol and can be provided in the form of a mousse, foam or a spray. When the cosmetic composition according to the invention are provided for protecting the human epidermis against UV radiation or as sunscreen composition, they can be in the form of a suspension or dispersion in solvents or fatty substances, or alternatively in the form of an emulsion or microemulsion (in particular of O/W or W/O type, O/W/O or W/O/W-type), such as a cream or a milk, a vesicular dispersion, in the form of an ointment, a gel, a solid tube stick or an aerosol mousse. The emulsions can also contain anionic, nonionic, cationic or amphoteric surfactants.

The following examples are provided to further illustrate the processes and compositions of the present invention. These examples are illustrative only and are not intended to limit the scope of the invention in any way.

Example 1: Determination of the water content by volumetric Karl Fischer titration

The water content of hydrothermal treated titanium dioxide and of a double coated titanium dioxide according to the invention has been determined by volumetric Karl Fischer (KF) titration with a Metrohm 758 KFD Titrino 703 TI Standard with a Double Pt-wire electrode, (Metrohm Art.: 6.0338.100) using Metrodata TiNet 2.4 software in methanol p.a. As KF reagent Hydranal Composite 5 (Riedel de Haen) was used.

**Calculation of the water equivalent (WE) of the titrant (Karl Fischer reagent):**

30-40 ml of methanol are placed into the titration flask. Afterwards the methanol is titrated to dryness with the KF reagent. Into the dry medium about 20 mg of water are added and titrated. The water equivalent is calculated according to:

$$WE\,[mg\,/ml] = \frac{\text{weight water}\,[mg]}{\text{volume of consumed KF reagent}\,[ml]}.$$

This is repeated 3 times and the mean of the results is used as WE.

**Sample determination:**

30-40 ml of methanol are placed into the titration flask. Afterwards the methanol is titrated to dryness with the KF reagent. Into the dry medium 150-250 mg of the analyt (i.e. titanium dioxide is added and titrated.
The water content is calculated according to:

$$\% \, water = \frac{\text{volume KF reagent consumed}\,[ml] \times WE\,[mg/ml]}{10 \times \text{weighted analyt}\,[g]}.$$

Table 1: water content

|  | Hydrothermal treated titanium dioxide coated with silica | Double coated titanium dioxide according to the invention |
|---|---|---|
| Water content | 2.16% | 1.2% |
| Dispersibility (Mygliol) | Moderate | Excellent |

As can be seen, the water content is significantly lower in the non-hydrothermal treated titanium dioxide whereas the wettability with a cosmetic oil is notably better leading to a better dispersibility.

Example 2: Photocatalytic activity

Untreated titanium dioxide produces an intense yellow coloration upon irradiation with UV-light. The more intense the color, the greater the reactivity of the titanium dioxide. This offers a good analytical test for the effectiveness of the coating.
A 10% dispersion of the coated $TiO_2$ in Caprylic/Capric Triglyceride in comparison to an uncoated sample and also in comparison to Uvinul® $TiO_2$. is drawn as a film on a glass plate with a 20 μm spreading knife. Afterwards irradiation was performed with a Heraeus Suntester with 40 MED. The judgment of the color of the samples was performed by comparison with "Methuen Handbook of Color", A. Kornerup und J.H. Wanscher, 3. edition, Eyre Methuen, London, 1984. Additionally, the samples were compared with untreated $TiO_2$ and with a commercially available single coated titanium dioxide grade: Uvinul® $TiO_2$ (octylsilylated titanium dioxide) and Eusolex® T Avo (silica coated titanium dioxide)

Table 2: Photocatalytic activity

| $TiO_2$ | Color* after irradiation with 40 MED |
|---|---|
| pyrogenic titanium dioxide (P25 ex Degussa) non treated | pastel yellow |
| Uvinul® $TiO_2$ Single, octylsilylated titanium dioxide | pale yellow |
| Eusolex® T Avo single, silica coated titanium dioxide | yellowish white |
| Double coated titanium dioxide according to the invention | white |
| *according to Table 3 "Methuen Handbook of Color", A. Kornerup und J.H. Wanscher, 3. edition. | |

As can be seen from the table2, the double coated grinded titanium dioxide grades showed a reduced coloration compared to the untreated $TiO_2$ and a better performance compared to the commercial avilable, single coated titanium dioxide grades Uvinul® $TiO_2$ (octylsilylated titanium) and Eusolex® T-Avo (silica) indicating a reduced photocatalytic activity.

Example 3: Increased in vivo SPF performance

Determination of the in vivo SPF according to COLIPA International Sun Protection Factor (SPF) Test Method, 2003 of an O/W emulsion as described below containing different titanium dioxide grades as well as 4,4'-Methoxy-tert.butyldibenzoylmethane (INCI: Butyl methoxydibenzoylmethane) and additional UV-filter in the indicated amounts.

Table 3: in vivo SPF

| **Ingredients** | **INCI Nomenclature** | **A** | **B** |
|---|---|---|---|
| PARSOL® 1789 | Butyl Methoxydibenzoylmethane | 4% | 4% |
| PARSOL® SLX | Polysilicone-15 | 3% | 3% |
| PARSOL® 340 | Octocrylene | 10% | 10% |
| Double coated titanium dioxide according to the invention | | 3% | |
| Uvinul® $TiO_2$ | Single coated titanium dioxide | | 3% |
| **In vivo SPF** | | **25** | **20** |

As can be seen from table 3 a significant increase in the in vivo SPF can be observed by using the double coated titanium dioxide according to the invention.

Example 4: Improvement of the Photostability of a dibenzoyl methane derivative

The film-photostability of 2% Butyl Methoxydibenzoyl methane (BMDBM) in combination with 5% of a double coated titanium dioxide in Cetiol A/ EtOH (30:70) irradiated with 10MED was measured according to G. Berset & H. Gonzenbach (COLIPA Task force); Proposed protocol for determination of photostability. Part I: cosmetic UV-filters, Int.J.Cosmet.Sci. 18, 167-177 (1996). As reference 2% Butyl Methoxydibenzoylmethane alone or in combination with a commercially available single coated titanium dioxide grade: Uvinul® $TiO_2$ (octylsilylated titanium dioxide from BASF) was used:

Table 4: Photostability of Butyl Methoxydibenzoylmethane

| Composition | Recovery of BMDBM (by UV) |
|---|---|
| 2% Butyl Methoxydibenzoylmethane | 8% |

(continued)

| Composition | Recovery of BMDBM (by UV) |
|---|---|
| 2% Butyl Methoxydibenzoylmethane, 5% double coated titanium dioxide according to the invention | 40% |
| 2% Butyl Methoxydibenzoylmethane, 5% Uvinul® TiO$_2$ | 5% |

As can bee seenfrom table 4, the double coated titanium dioxide according to the invention with a water content < 1.5% significantly enhances the photostability of Butyl Methoxydibenzoylmethane.

Example 5: O/W sun milk

| | Ingredients | INCI Nomenclature | % w / w |
|---|---|---|---|
| A) | PARSOL® SLX | Polysilicone-15 | 6.00 |
| | PARSOL® 1789 | Butyl Methoxydibenzoylmethane | 2.00 |
| | Lanette O | Cetearyl Alcohol | 2.00 |
| | Myritol 318 | Caprylic/capric Triglyceride | 6.00 |
| | Mineral oil | Mineral oil | 2.00 |
| | Vitamin E acetate | Tocopheryl Acetate | 1.00 |
| | Prisorine 3515 | Isostearyl Alcohol | 4.00 |
| B) | Edeta BD | Disodium EDTA | 0.10 |
| | Neo Heliopan® AP | | 3.00 |
| | Phenonip | Phenoxyethanol & Methylparaben & Ethylparaben & Propylparaben & Butylparaben | 0.60 |
| | Amphisol K | Potassium Cetyl Phosphate | 2.00 |
| | Water deionized | Aqua | ad100 |
| | 1,2-Propylen Glycol | Propylene Glycol | 5.00 |
| | Carbopol 981 | Carbomer | 0.30 |
| | Tinosorb® M | Methylene Bis-Benzotriazolyl Tetramethylbutylphenol | 6.00 |
| | KOH 10% solution | Potassium Hydroxyde | 2.10 |
| C) | 'Double coated titanium dioxide' | | 0.01-25 |

Procedure:

Heat part A)and B) to 85°C while stirring. When homogeneous, add part B) to A) under agitation. Cool to ambient temperature while stirring and add part C). Homogenize to achieve a small particle size.

Example 6: O/W sun milk

| | Ingredients | INCI Nomenclature | % w / w |
|---|---|---|---|
| A) | PARSOL® SLX | Polysilicone-15 | 3.00 |
| | Uvinul® A Plus | Diethylamino Hydroxybenzoyl Hexyl Benzoate | 2.00 |
| | Lanette O | Cetearyl Alcohol | 2.00 |
| | Myritol 318 | Caprylic/capric Triglyceride | 6.00 |
| | Mineral oil | Mineral oil | 2.00 |
| | Vitamin E acetate | Tocopheryl Acetate | 1.00 |
| | Prisorine 3515 | Isostearyl Alcohol | 4.00 |

(continued)

|  | Ingredients | INCI Nomenclature | % w / w |
|---|---|---|---|
| B) | Edeta BD | Disodium EDTA | 0.10 |
|  | Neo Heliopan® AP |  | 3.00 |
|  | Phenonip | Phenoxyethanol & Methylparaben & Ethylparaben & Propylparaben & Butylparaben | 0.60 |
|  | Amphisol K | Potassium Cetyl Phosphate | 2.00 |
|  | Water deionized | Aqua | ad100 |
|  | 1,2-Propylen Glycol | Propylene Glycol | 5.00 |
|  | Carbopol 981 | Carbomer | 0.30 |
|  | Tinosorb® M | Methylene Bis-Benzotriazolyl Tetramethylbutylphenol | 6.00 |
|  | KOH 10% solution | Potassium Hydroxyde | 2.10 |
| C) | 'Double coated titanium dioxide' |  | 0.01-25 |

Procedure:

Heat part A)and B) to 85°C while stirring. When homogeneous, add part B) to A) under agitation. Cool to ambient temperature while stirring and add part C). Homogenize to achieve a small particle size.

Example 7: Sun milk waterproofed

|  | Ingredients | INCI Nomenclature | % w / w |
|---|---|---|---|
| A) | PARSOL® SLX | Polysilicone-15 | 3.00 |
|  | PARSOL® 1789 | Butyl Methoxydibenzoylmethane | 2.00 |
|  | PARSOL® 5000 | 4-Methylbenzylidene Camphor | 4.00 |
|  | Uvinul® T 150 | Ethylhexyltriazone | 2.00 |
|  | Silicone DC 200/350 cs | Dimethicone | 1.00 |
|  | Lanette O | Cetearyl Alcohol | 2.00 |
|  | Softisan 100 | Hydrogenated Coco-Glycerides | 3.00 |
|  | Tegosoft TN | C12-15 Alkyl Benzoate | 6.00 |
|  | Cetiol B | Dibutyl Adipate | 7.00 |
|  | Vitamin E acetate | Tocopheryl Acetate | 2.00 |
|  | BHT | BHT | 0.05 |
|  | Edeta BD | Disodium EDTA | 0.10 |
|  | Phenonip | Phenoxyethanol & Methylparaben & Ethylparaben & Propylparaben & Butylparaben | 1.00 |
|  | Amphisol® K | Cetyl Phosphate potassium | 2.00 |
| B) | Water deionized | Aqua | ad 100 |
|  | Propylene Glycol | Propylene Glycol | 5.00 |
|  | Carbopol 980 | Carbomer | 0.30 |
|  | KOH (10% sol.) | Potassium Hydroxide | 1.50 |
| C) | 'Double coated titanium dioxide' |  | 0.01-25 |

Procedure:

Heat part A)and B) to 85°C while stirring. When homogeneous, add part B) to A) under agitation. Cool to ambient temperature while stirring and add part C). Homogenize to achieve a small particle size.

Example 8 :High SPF sun milk

| | | Ingredients | INCI Nomenclature | % w / w |
|---|---|---|---|---|
| A) | | PARSOL® SLX | Polysilicone-15 | 3.00 |
| | | PARSOL® 1789 | Butyl Methoxydibenzoylmethane | 2.00 |
| | | PARSOL® 5000 | 4-Methylbenzylidene Camphor | 4.00 |
| | | Uvinul® T 150 | Octyl Triazone | 2.00 |
| | | Silicone DC 200/350 cs | Dimethicone | 1.00 |
| | | Lanette O | Cetearyl Alcohol | 2.00 |
| | | Softisan 100 | Hydrogenated Coco-Glycerides | 3.00 |
| | | Tegosoft TN | C12-15 Alkyl Benzoate | 6.00 |
| | | Cetiol B | Dibutyl Adipate | 7.00 |
| | | Vitamin E acetate | Tocopheryl Acetate | 2.00 |
| | | BHT | BHT | 0.05 |
| | | Edeta BD | Disodium EDTA | 0.10 |
| | | Phenonip | Phenoxyethanol & Methylparaben & Ethylparaben & Propylparaben & Butylparaben | 0.60 |
| | | Amphisol® K | Potassium Cetyl Phosphate | 2.00 |
| B) | | Water deionized | Aqua | ad 100 |
| | | Propylene Glycol | Propylene Glycol | 5.00 |
| | | Carbopol 980 | Carbomer | 0.30 |
| | | KOH (10% sol.) | Potassium Hydroxide | 1.50 |
| C) | | 'Double coated titanium dioxide' | | 0.01-25 |
| D) | | Perfume | Perfume | q.s. |

Procedure:

Heat part A)and B) to 85°C while stirring. When homogeneous, add part B) to A) under agitation. Cool to ambient temperature while stirring and add part C) and D). Homogenize to achieve a small particle size.

Example 9: Water-free sun gel

| | | Ingredients | INCI Nomenclature | % w / w |
|---|---|---|---|---|
| A) | | PARSOL® MCX | Ethylhexyl Methoxycinnamate | 6.00 |
| | | PARSOL® 1789 | Butyl Methoxydibenzoylmethane | 4.00 |
| | | PARSOL® 5000 | 4-Methylbenzylidene Camphor | 4.00 |
| | | Uvasorb® HEB | Diethylhexyl Butamido Triazone | 1.50 |
| | | Vitamin E acetate | Tocopheryl Acetate | 1.50 |
| | | Tegosoft TN | C12-15 Alkyl Benzoate | 9.00 |
| | | Elefac I-205 | Ethylhexyldodecyl Neopentanoate | 2.00 |
| | | Alcohol | Alcohol | ad 100 |
| | | Isopropyl Alcohol | Isopropyl Alcohol | 20.00 |
| B) | | Klucel MF | Hydroxypropylcellulose | 2.00 |
| C) | | Double coated titanium dioxide' | | 0.01-25 |
| D) | | perfume | | q.s. |

Procedure:

Mix part A) and B) while stirring. When homogeneous, add part C) and D) under agitation.

Example 10: Sun gel

|   | Ingredients | INCI Nomenclature | % w / w |
|---|---|---|---|
| A) | Pemulen TR-2 | Acrylates/C10-30 Alky Acrylate Crosspolymer | 0.60 |
|   | Phenonip | Phenoxyethanol & Methylparaben & Ethylparaben & Propylparaben & Butylparaben | 0.60 |
|   | Edeta BD | Disodium EDTA | 0.1 |
|   | Aqua | Aqua | ad 100 |
| B) | PARSOL® 1789 | Butyl Methoxydibenzoylmethane | 4.00 |
|   | PARSOL® 340 | Octocrylene | 3.00 |
|   | Tegosoft TN | C12-15 Alkyl Benzoate | 15.00 |
|   | Antaron V-216 | PVP/Hexadecene Copolymer | 1.00 |
|   | Vitamin E acetate | Tocopheryl Acetate | 0.50 |
|   | Butylated Hydroxytoluene | BHT | 0.05 |
|   | Cremophor RH 410 | PEG-40 Hydrogenated Castor Oil | 0.50 |
|   | Tris Amino | Tromethamine | 0.50 |
| C) | Double coated titanium dioxide' |   | 0.01-25 |
| D) | Perfume | Perfume | q.s. |

Procedure:

Heat part A)and B) to 85°C while stirring. When homogeneous, add part B) to A) under agitation. Cool to ambient temperature while stirring and add part C) and D). Homogenize to achieve a small particle size.

Example 11: High protection W/O sun milk

|   | Ingredients | INCI Nomenclature | % w / w |
|---|---|---|---|
| A) | PARSOL® 1789 | Butyl Methoxydibenzoylmethane | 2.00 |
|   | Uvinul® T 150 | Ethylhexyl Triazone | 2.00 |
|   | Arlacel P 135 | PEG-30 Dipolyhydroxystearate | 2.00 |
|   | Tegosoft TN | C12-15 Alkyl Benzoate | 5.00 |
|   | Cosmacol EMI | Di-C12-13 Alkyl Malate | 6.00 |
|   | Miglyol 840 | Propylene Glycol Dicaprylate/Dicaprate | 6.00 |
|   | Butylated Hydroxytoluene | BHT | 0.05 |
|   | Phenonip | Phenoxyethanol & Methylparaben & Ethylparaben & Propylparaben & Butylparaben | 0.60 |
| B) | Deionized water | Aqua | ad 100 |
|   | Glycerin | Glycerin | 5.00 |
|   | Edeta | Disodium EDTA | 0.1 |
|   | NaCl | Sodium Chloride | 0.30 |
| C) | PARSOL® HS | Phenylbenzyimidazole Sulphonic Acid | 4.00 |
|   | Water | Aqua | 20.00 |

(continued)

| | Ingredients | INCI Nomenclature | % w / w |
|---|---|---|---|
| | Triethanolamine 99%. | Triethanolamine | 2.50 |
| D) | Double coated titanium dioxide' | | 0.01-25 |
| E) | Perfume | | q.s. |

Procedure:

Heat part A), B) and C) to 85°C while stirring. When homogeneous, add part B) and C) to A) under agitation. Cool to ambient temperature while stirring and add part D) and E). Homogenize to achieve a small particle size.

Example 12: W/O milk with pigments

| | Ingredients | INCI Nomenclature | % w / w |
|---|---|---|---|
| A) | Cremophor WO 7 | PEG-7 Hydrogenated Castor Oil | 6.00 |
| | Elfacos ST 9 | PEG-45/Dodecyl Glycol Copolymer | 2.00 |
| | PARSOL® 1789 | Butyl Methoxydibenzoylmethane | 3.00 |
| | Tinosorb® S | Bis-Ethylhexylocxyphenol Methoxyphenol Trazine | 5.00 |
| | PARSOL® 5000 | 4-Methylbenzylidene Camphor | 4.00 |
| | microfine ZnO | Zinc Oxide | 2.00 |
| | Microcrystalline wax | Microcrystalline Wax | 2.00 |
| | Miglyol 812 | Caprylic/capric Triglyceride | 5.00 |
| | Vitamin E acetate | Tocopheryl Acetate | 1.00 |
| | Jojoba oil | Simmondsia Chinensis Seed Oil | 5.00 |
| | Edeta BD | Disodium EDTA | 0.10 |
| | Butylated Hydroxytoluene | BHT | 0.05 |
| | Phenonip | Phenoxyethanol & Methylparaben & Ethylparaben & Propylparaben & Butylparaben | 0.60 |
| B) | Water deionized | Aqua | ad 100 |
| | Glycerin | Glycerin | 5.00 |
| C) | Neo Heliopan® AP | | 2.00 |
| | Water deionized | Aqua | 20.00 |
| | KOH 10% solution | Potassium Hydroxide | 4.00 |
| D) | Double coated titanium dioxide' | | 0.01-25 |
| E) | Perfume | Perfume | q.s. |

Procedure:

Heat part A), B) and C) to 85°C while stirring. When homogeneous, add part B) and C) to A) under agitation. Cool to ambient temperature while stirring and add part D) and E). Homogenize to achieve a small particle size.

Example 13: Protective Day cream with Vitamin C

| | Ingredients | INCI Nomenclature | % w / w |
|---|---|---|---|
| A) | PARSOL® SLX | Polysilicone-15 | 2.50 |

(continued)

|  | Ingredients | INCI Nomenclature | % w / w |
|---|---|---|---|
|  | PARSOL® 1789 | Butyl Methoxydibenzoylmethane | 1.50 |
|  | Glyceryl Myristate | Glyceryl Myristate | 2.00 |
|  | Cetyl Alcohol | Cetyl Alcohol | 0.50 |
|  | Myritol 318 | Caprylic/Capric Triglyceride | 5.00 |
|  | Crodamol DA | Diisopropyl Adipate | 5.00 |
|  | Vitamin E acetate | Tocopheryl Acetate | 2.00 |
|  | Butylated Hydroxytoluene | BHT | 0.05 |
|  | Phenonip | Phenoxyethanol & Methylparaben & 0.60 Ethylparaben & Propylparaben & Butylparaben |  |
|  | Edeta BD | Disodium EDTA | 0.10 |
|  | Amphisol® K | Potassium Cetyl Phosphate | 2.00 |
| B) | Water deionized | Aqua | ad 100 |
|  | 1,2-Propylene Glycol | Propylene Glycol | 2.00 |
|  | D-Panthenol 75 L | Panthenol | 2.00 |
|  | Ethanol | Ethanol | 5.00 |
|  | Allantoin | Allantoin | 0.20 |
|  | Carbopol ETD 2001 | Carbomer | 0.30 |
|  | KOH 10% sol. | Potassium Hydroxide | 1.50 |
| C) | Water | Aqua | 10.00 |
|  | Stay-C 50 | Sodium Ascorbyl Phosphate | 0.50 |
| D) | Double coated titanium dioxide' |  | 0.01-25 |
| E) | Perfume | Perfume | q.s. |

Example 14: Photostable O/W sun milk

|  | Ingredients | INCI Nomenclature | % w / w |
|---|---|---|---|
| A) | PARSOL® 1789 | Butyl Methoxydibenzoylmethane | 2.00 |
|  | PARSOL® 340 | Octocrylene | 1.80 |
|  | PARSOL® SLX | Polysilicone-15 | 3.00 |
|  | Lanette O | Cetearyl Alcohol | 2.00 |
|  | Myritol 318 | Caprylic/capric Triglyceride | 6.00 |
|  | Mineral oil | Mineral oil | 2.00 |
|  | Vitamin E acetate | Tocopheryl Acetate | 1.00 |
|  | Prisorine 3515 | Isostearyl Alcohol | 4.00 |
| B) | Edeta BD | Disodium EDTA | 0.10 |
|  | Phenonip | Phenoxyethanol & Methylparaben & Ethylparaben & Propylparaben & Butylparaben | 0.60 |
|  | Amphisol® K | Potassium Cetyl Phosphate | 2.00 |
|  | Water deionized | Aqua | ad100 |
|  | 1,2-Propylen Glycol | Propylene Glycol | 5.00 |
|  | Carbopol 981 | Carbomer | 0.30 |
|  | KOH 10% solution | Potassium Hydroxyde | 2.10 |
| C) | 'Double coated titanium dioxide' |  | 0.01-25 |

Procedure:

Heat part A) and B) to 85°C while stirring. When homogeneous, add part B) to A) under agitation. Cool to ambient temperature while stirring and add part C). Homogenize to achieve a small particle size.

**Claims**

1.  Cosmetic or dermatological compositions comprising multiply coated titanium dioxide particles, said particles having at least one inner inorganic silica coating and one outer organic coating and a water content of less than 1.5 %.

2.  Cosmetic or dermatological compositions according to claim 1 comprising additionally a dibenzoyl methane derivative.

3.  Cosmetic or dermatological compositions according to claim 1 or 2, wherein in the multiply coated titanium dioxide particles the outer coating is selected from silicone oils, alkyl silanes, olefinic acids, polyols or organophosphonic acids and mixtures thereof.

4.  Cosmetic or dermatological compositions to any one of claims 1 to 3, wherein in the multiply coated titanium dioxide particles the outer coating is selected from simethicone, methicone, dimethicone, polysilicone-15, stearic acid, glycerol and mixtures thereof.

5.  Cosmetic or dermatological compositions according to any one of claims 1 to 4, wherein in the multiply coated titanium dioxide particles the outer coating is selected from methicone, dimethicone, polysilicone-15 or stearic acid.

6.  Cosmetic or dermatological compositions according to any one of claims 1 to 5 wherein the outer coating of the titanium dioxide is dimethicone.

7.  Cosmetic or dermatological compositions according to any one of claims 2 to 6 wherein the dibenzoyl methane derivative is 4,4'-Methoxy-tert.butyldibenzoylmethane.

8.  Cosmetic or dermatological composition according to any of claims 2 to 7 comprising from 1-25 wt.-% of the multiply coated titanium dioxide and 0.5-7 wt.-% of the dibenzoylmethane derivative based on the total weight of the composition.

9.  Cosmetic or dermatological composition according to any one of claims 1 to 8 wherein further UV-A screening agent/s and/or UV-B screening agent/s and/ or broadband screening agent/s are additionally present.

10. Cosmetic or dermatological composition according to any one of claims 2 to 9 wherein a photostabilizer is additionally present.

11. Cosmetic or dermatological composition according to claim 10 wherein the photostabilizer is selected from polysilicone-15, octocrylene, 4-methylbenzylidene camphor or mixtures thereof.

12. The use of the multiply coated titanium dioxide particles having at least one inner inorganic silica coating and one outer organic coating, and wherein the titanium dioxide has a water content of less than 1.5%, optionally in combination with an additional photostabilizer such as octocrylene, polysilicone-15, 4-methylbenzylidene camphor or mixtures thereof, for the stabilization of a dibenzoyl methane or a derivative thereof.

13. The use of multiply coated titanium dioxide particles having at least one inner inorganic silica coating and one outer organic coating, and wherein the titanium dioxide has a water content of less than 1.5%, for the enhancement of the sun protection factor (SPF) of cosmetic and dermatological compositions containing a dibenzoyl methane derivative.

**Patentansprüche**

1.  Kosmetische oder dermatologische Zusammensetzungen, umfassend mehrfach beschichtete Titandioxidteilchen, wobei die Teilchen mindestens eine innere anorganische Siliciumdioxidbeschichtung und eine äußere organische Beschichtung und einen Wassergehalt von weniger als 1,5% aufweisen.

2. Kosmetische oder dermatologische Zusammensetzungen nach Anspruch 1, die zusätzlich ein Dibenzoylmethanderivat umfassen.

3. Kosmetische oder dermatologische Zusammensetzungen nach Anspruch 1 oder 2, wobei in den mehrfach beschichteten Titandioxidteilchen die äußere Beschichtung aus Silikonölen, Alkylsilanen, olefinischen Säuren, Polyolen oder Organophosphonsäuren und Mischungen davon ausgewählt ist.

4. Kosmetische oder dermatologische Zusammensetzungen nach einem der Ansprüche 1 bis 3, wobei in den mehrfach beschichteten Titandioxidteilchen die äußere Beschichtung aus Simethicon, Methicon, Dimethicon, Polysilikon-15, Stearinsäure, Glycerin und Mischungen davon ausgewählt ist.

5. Kosmetische oder dermatologische Zusammensetzungen nach einem der Ansprüche 1 bis 4, wobei in den mehrfach beschichteten Titandioxidteilchen die äußere Beschichtung aus Methicon, Dimethicon, Polysilikon-15 oder Stearinsäure ausgewählt ist.

6. Kosmetische oder dermatologische Zusammensetzungen nach einem der Ansprüche 1 bis 5, wobei es sich bei der äußeren Beschichtung des Titandioxids um Dimethicon handelt.

7. Kosmetische oder dermatologische Zusammensetzungen nach einem der Ansprüche 2 bis 6, wobei es sich bei dem Dibenzoylmethanderivat um 4,4'-Methoxytert.-butyldibenzoylmethan handelt.

8. Kosmetische oder dermatologische Zusammensetzung nach einem der Ansprüche 2 bis 7, umfassend 1-25 Gew.-% des mehrfach beschichteten Titandioxids und 0,5-7 Gew.-% des Dibenzoylmethanderivats, bezogen auf das Gesamtgewicht der Zusammensetzung.

9. Kosmetische oder dermatologische Zusammensetzung nach einem der Ansprüche 1 bis 8, wobei zusätzlich weitere UV-A-Filtersubstanzen und/oder UV-B-Filtersubstanzen und/oder Breitband-Filtersubstanzen vorliegen.

10. Kosmetische oder dermatologische Zusammensetzung nach einem der Ansprüche 2 bis 9, wobei zusätzlich ein Lichtstabilisator vorliegt.

11. Kosmetische oder dermatologische Zusammensetzung nach Anspruch 10, wobei der Lichtstabilisator aus Polysilikon-15, Octocrylen, 4-Methylbenzylidencampher oder Mischungen davon ausgewählt ist.

12. Verwendung der mehrfach beschichteten Titandioxidteilchen mit mindestens einer inneren anorganischen Siliciumdioxidbeschichtung und einer äußeren organischen Beschichtung und wobei das Titandioxid einen Wassergehalt von weniger als 1,5% aufweist, gegebenenfalls in Kombination mit einem zusätzlichen Lichtstabilisator wie Octocrylen, Polysilikon-15, 4-Methylbenzylidencampher oder Mischungen davon, zur Stabilisierung eines Dibenzoylmethans oder eines Derivats davon.

13. Verwendung der mehrfach beschichteten Titandioxidteilchen mit mindestens einer inneren anorganischen Siliciumdioxidbeschichtung und einer äußeren organischen Beschichtung und wobei das Titandioxid einen Wassergehalt von weniger als 1,5% aufweist, zur Erhöhung des Lichtschutzfaktors (LSF) von kosmetischen und dermatologischen Zusammensetzungen, die ein Dibenzoylmethanderivat enthalten.

**Revendications**

1. Compositions cosmétiques ou dermatologiques comprenant des particules de dioxyde de titane à revêtements multiples, lesdites particules ayant au moins un revêtement de silice inorganique interne et un revêtement organique externe, et une teneur en eau inférieure à 1,5%.

2. Compositions cosmétiques ou dermatologiques selon la revendication 1, comprenant en outre un dérivé de dibenzoylméthane.

3. Compositions cosmétiques ou dermatologiques selon la revendication 1 ou 2, dans lesquelles, dans les particules de dioxyde de titane à revêtements multiples, le revêtement externe est choisi parmi les huiles siliconées, les alkylsilanes, les acides oléfiniques, les polyols ou les acides organophosphoriques, et des mélanges de ceux-ci.

4. Compositions cosmétiques ou dermatologiques selon l'une quelconque des revendications 1 à 3, dans lesquelles, dans les particules de dioxyde de titane à revêtements multiples, le revêtement externe est choisi parmi la siméthicone, la méthicone, la diméthicone, le polysilicone-15, l'acide stéarique, le glycérol et des mélanges de ceux-ci.

5. Compositions cosmétiques ou dermatologiques selon l'une quelconque des revendications 1 à 4, dans lesquelles, dans les particules de dioxyde de titane à revêtements multiples, le revêtement externe est choisi parmi la méthicone, la diméthicone, le polysilicone-15 ou l'acide stéarique.

6. Compositions cosmétiques ou dermatologiques selon l'une quelconque des revendications 1 à 5, dans lesquelles le revêtement externe du dioxyde de titane est la diméthicone.

7. Compositions cosmétiques ou dermatologiques selon l'une quelconque des revendications 2 à 6, dans lesquelles le dérivé de dibenzoylméthane est le 4,4'-méthoxy-tertio-butyldibenzoylméthane.

8. Composition cosmétique ou dermatologique selon l'une quelconque des revendications 2 à 7, comprenant 1-25% en poids de dioxyde de titane à revêtements multiples et 0,5-7% en poids du dérivé de dibenzoylméthane, sur la base du poids total de la composition.

9. Composition cosmétique ou dermatologique selon l'une quelconque des revendications 1 à 8, dans laquelle un ou plusieurs autres agents de filtre anti-UV A et/ou agents de filtre anti-UV B et/ou agents de filtre à large bande, sont en outre présents.

10. Composition cosmétique ou dermatologique selon l'une quelconque des revendications 2 à 9, dans laquelle un agent photostabilisant est en outre présent.

11. Composition cosmétique ou dermatologique selon la revendication 10, dans laquelle l'agent photostabilisant est choisi parmi le polysilicone-15, l'octocrylène, le 4-méthylbenzylidènecamphre, ou des mélanges de ceux-ci.

12. Utilisation des particules de dioxyde de titane à revêtements multiples, ayant au moins un revêtement de silice inorganique interne et un revêtement organique externe, et dans laquelle le dioxyde de titane possède une teneur en eau inférieure à 1,5%, éventuellement en combinaison avec un autre agent photostabilisant tel que l'octocrylène, le polysilicone-15, le 4-méthylbenzylidènecamphre, ou des mélanges de ceux-ci, pour la stabilisation d'un dibenzoylméthane ou d'un dérivé de celui-ci.

13. Utilisation de particules de dioxyde de titane à revêtements multiples, ayant au moins un revêtement de silice inorganique interne et un revêtement organique externe, et dans laquelle le dioxyde de titane possède une teneur en eau inférieure à 1,5%, pour l'amélioration du facteur de protection solaire (FPS) de compositions cosmétiques et dermatologiques contenant un dérivé de dibenzoylméthane.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- FR 2326405 A
- FR 2440933 A
- EP 0114607 A
- EP 1281388 A
- EP 988853 A
- EP 1284277 A
- EP 0988853 A
- US 5562897 A
- JP 2000319128 B
- EP 748624 A
- WO 0222098 A
- DE 10333029 A1

- EP 444798 A
- EP 44515 A
- FI 57124
- EP 0895776 A
- EP 0358584 B1
- EP 0538431 B1
- EP 0709080 A1
- EP 1046391 A
- EP 0514491 B1
- EP 0780119 A1
- US 5605680 A

### Non-patent literature cited in the description

- **EUGEN SCHOLZ.** Karl-Fischer-Titration. Springer-Verlag, 1984
- **NOVAK G.A.** *Die kosmetischen Präparate - Band 2, Die kosmetischen Präparate - Rezeptur, Rohstoffe,* vol. 2
- **A. KORNERUP ; J.H. WANSCHER.** Methuen Handbook of Color. 1984

- **A. KORNERUP ; J.H. WANSCHER.** Methuen Handbook of Color
- **G. BERSET ; H. GONZENBACH.** COLIPA Task force); Proposed protocol for determination of photostability. Part I: cosmetic UV-filters. *Int.J.Cosmet.Sci.,* 1996, vol. 18, 167-177